# EUROPÄISCHE PATENTANMELDUNG

(11) **EP 4 421 817 A1**
(43) Veröffentlichungstag der Anmeldung: **28.08.2024**
(21) Anmeldenummer: 23158555.5
(22) Anmeldetag: 24.02.2023
(51) Int. Cl.: G16H 20/17, G16H 40/40, G16H 40/63, A61M 5/168

(54) **VERFAHREN ZUM BETREIBEN EINES INFUSIONSSYSTEMS UND INFUSIONSSYSTEM**

(71) Anmelder: B. Braun Melsungen AG, 34212 Melsungen (DE)
(72) Erfinder: Obermann, Dennis, 34628 Willingshausen (DE); Wiegand, Thomas, 34576 Homberg (DE); Moritzen, Hans-Christian, 34119 Kassel (DE)
(74) Vertreter: Patentanwälte Ruff, Wilhelm, Beier, Dauster & Partner mbB

(57) **Zusammenfassung**

Verfahren zum Betreiben eines Infusionssystems (1) mit den Schritten:
- Fördern einer zu verabreichenden Flüssigkeit (3) in einer Flüssigkeitsleitung (4) mit einem vorgebbaren Volumenstrom,
- fortlaufendes Überwachen, ob ein Verschluss (V) der Flüssigkeitsleitung (4) vorliegt,
- falls ein Verschluss der Flüssigkeitsleitung (4) vorliegt, fortlaufendes Berechnen eines aufgrund des Verschlusses (V) der Flüssigkeitsleitung (4) nicht geförderten Fehlvolumens der zu verabreichenden Flüssigkeit (3) und/oder einer daraus abgeleiteten Größe, und
- Ausgeben eines Alarms, sobald das Fehlvolumen der zu verabreichenden Flüssigkeit (3) und/oder die daraus abgeleitete Größe einen einstellbaren Fehlvolumenschwellenwert und/oder einen daraus abgeleiteten Schwellenwert überschreitet.

## Beschreibung

Der Erfindung liegt die Aufgabe zugrunde, ein Verfahren zum Betreiben eines Infusionssystems und ein Infusionssystem zur Verfügung zu stellen, die bei einem Verschluss einer Flüssigkeitsleitung des Infusionssystems eine möglichst zuverlässige Alarmierung ermöglichen.

Das Verfahren dient zum Betreiben eines medizinischen Infusionssystems.

Das Verfahren weist die Schritte auf: Fördern einer einem Patienten zu verabreichenden Flüssigkeit in einer Flüssigkeitsleitung mit einem vorgebbaren bzw. bekannten Volumenstrom, kontinuierliches Überwachen, ob ein unerwünschter Verschluss der Flüssigkeitsleitung vorliegt, falls ein Verschluss der Flüssigkeitsleitung vorliegt, kontinuierliches Berechnen eines aufgrund des Verschlusses der Flüssigkeitsleitung nicht geförderten Fehlvolumens der zu verabreichenden Flüssigkeit und/oder einer daraus abgeleiteten Größe, und Ausgeben eines Alarms, sobald das Fehlvolumen der zu verabreichenden Flüssigkeit und/oder die daraus abgeleitete Größe einen einstellbaren Fehlvolumenschwellenwert und/oder einen daraus abgeleiteten Schwellenwert überschreitet. Der Volumenstrom kann auch als Förderrate bezeichnet werden. Die zu verabreichende Flüssigkeit ist typisch ein Medikament bzw. enthält ein Medikament.

In einer Ausführungsform wird/werden der Fehlvolumenschwellenwert und/oder der daraus abgeleitete Schwellenwert in Abhängigkeit von der Art der zu verabreichenden Flüssigkeit automatisch eingestellt. Beispielsweise werden bei Flüssigkeiten bzw. Medikamenten, die in kleinen Volumenströmen bzw. Volumina verabreicht werden, geringere Fehlvolumenschwellenwerte automatisch eingestellt als bei Flüssigkeiten bzw. Medikamenten, die in größeren Volumenströmen bzw. Volumina verabreicht werden.

In einer Ausführungsform wird die zu verabreichende Flüssigkeit aus einem Einmalartikel gefördert, wobei der Fehlvolumenschwellenwert und/oder der daraus abgeleitete Schwellenwert automatisch eimalartikelspezifisch eingestellt wird/werden. Beispielsweise werden bei Einmalartikeln mit geringeren darin enthaltenen Flüssigkeits-Volumina geringere Fehlvolumenschwellenwerte automatisch eingestellt als bei Einmalartikeln mit größeren darin enthaltenen Flüssigkeits-Volumina.

In einer Ausführungsform wird/werden der Fehlvolumenschwellenwert und/oder der daraus abgeleitete Schwellenwert in Abhängigkeit von einer mit der zu verabreichenden Flüssigkeit korrespondierenden Therapieform, insbesondere automatisch, eingestellt. Beispielsweise wird bei Therapieformen mit typisch höheren Volumenströmen der zu verabreichenden Flüssigkeit ein höherer Fehlvolumenschwellenwert eingestellt als bei Therapieformen mit typisch geringeren Volumenströmen der zu verabreichenden Flüssigkeit.

In einer Ausführungsform wird/werden der Fehlvolumenschwellenwert und/oder der daraus abgeleitete Schwellenwert aus einer Medikamentendatenbank ausgelesen.

In einer Ausführungsform weist das fortlaufende Überwachen, ob ein Verschluss der Flüssigkeitsleitung vorliegt, die Schritte auf: Ermitteln einer Anzahl von Sensordaten, Speisen einer Kl-Einheit mit der Anzahl von Sensordaten, und mittels der KI-Einheit und basierend auf der Anzahl von Sensordaten Bestimmen, ob ein Verschluss der Flüssigkeitsleitung vorliegt. Mittels der der KI-Einheit können die die relevanten Sensordaten, beispielswiese betreffend einen mittels der Pumpe erzeugten Druck und/oder eine mittels der Pumpe erzeugte Pump-Kraft, und gegebenenfalls weitere Daten bzw. Eingangsgrößen ausgewertet werden, die auf einen Verschluss schließen lassen. Erfindungsgemäß werden diese Eingangsgrößen über eine Mustererkennung zur Verschlusserkennung ausgewertet. Die KI-Einheit kann integrierter Bestandteil einer Infusionspumpe sein. Die KI-Einheit kann einen nicht-selbstlernenden KI-Algorithmus ausführen.

In einer Ausführungsform enthält die Anzahl von Sensordaten: Sensordaten in Form von Druckdaten der zu verabreichenden Flüssigkeit in der Flüssigkeitsleitung, und/oder Sensordaten in Form von Kraftdaten einer mittels einer Infusionspumpe des Infusionssystems erzeugten Pumpkraft, und/oder Sensordaten in Form von Motorstromdaten eines elektrischen Antriebsmotors der Infusionspumpe, und/oder Sensordaten in Form von Positionsdaten eines Spritzenkolbens der Infusionspumpe.

Das erfindungsgemäße Infusionssystem weist auf: Mittel zum Fördern einer zu verabreichenden Flüssigkeit in einer Flüssigkeitsleitung mit einem vorgebbaren Volumenstrom, insbesondere in Form einer steuer- bzw. regelbaren Infusionspumpe, Mittel zum fortlaufenden Überwachen, ob ein Verschluss der Flüssigkeitsleitung vorliegt, insbesondere in Form einer KI-Einheit, und eine Steuereinrichtung, die dazu ausgebildet ist, falls ein Verschluss der Flüssigkeitsleitung vorliegt, ein aufgrund des Verschlusses der Flüssigkeitsleitung nicht gefördertes Fehlvolumen der zu verabreichenden Flüssigkeit und/oder eine daraus abgeleitete Größe fortlaufend zu berechnen, und einen Alarm auszugeben, sobald das Fehlvolumen der zu verabreichenden Flüssigkeit und/oder die daraus abgeleitete Größe einen einstellbaren Fehlvolumenschwellenwert und/oder einen daraus abgeleiteten Schwellenwert überschreitet. Die Ausgabe des Alarms kann beispielsweise optisch, akustisch, haptisch und/oder datenbasiert erfolgen, indem beispielsweise über ein Datennetzwerk Alarmdaten übertragen werden, wobei diese Alarmdaten von einem Empfänger entsprechend verarbeitet werden.

In einer Ausführungsform weist das Infusionssystem mindestens einen Sensor der folgenden Sensoren auf: Drucksensoren, die dazu ausgebildet sind, Sensordaten in Form von Druckdaten der zu verabreichenden Flüssigkeit in der Flüssigkeitsleitung zu erzeugen, Kraftsensoren, die dazu ausgebildet sind, Sensordaten in Form von Kraftdaten einer mittels der Infusionspumpe erzeugten Pumpkraft zu erzeugen, Motorstromsensoren, die dazu ausgebildet sind, Sensordaten in Form von Motorstromdaten eines elektrischen Antriebsmotors der Infusionspumpe zu erzeugen, und Positionssensoren, die dazu ausgebildet sind, Sensordaten in Form von Positionsdaten eines Spritzenkolbens der Infusionspumpe zu erzeugen.

Ziel einer Infusionstherapie ist eine kontinuierliche, über eine bestimmte Zeit ablaufende Verabreichung von Flüssigkeiten. Dies geschieht in der Regel intravenös, arteriell, subkutan oder intraossär über einen Katheter. Daneben gibt es noch die parenterale Ernährung mittels Infusionspumpen.

Für eine hohe Dosiergenauigkeit werden in der apparativen Infusionstechnik Infusionspumpen verwendet. Bekannte Ausprägungen von Infusionspumpen sind beispielsweise Spritzenpumpen sowie Schlauchpumpen. Während der Anwendung mit diesen Pumpen kann es zu verschiedenen Betriebsstörungen kommen. Ein Beispiel ist der Verschluss der Flüssigkeitsleitung und/oder des Patientenzugangs. Diese Betriebsstörung wird auch als Okklusion bezeichnet und führt zu einer Unterbrechung der Förderung. Da dies zu einer erheblichen Patientengefährdung führen kann, ist die Detektion eines Verschlusses fester Funktionsumfang herkömmlicher Infusionsgeräte.

Herkömmliche Infusionssysteme verwenden üblicherweise feste Druck-Schwellwerte, um einen Verschluss zu detektieren. Bei kleinsten Volumenströmen bzw. Infusionsraten kann es hierbei jedoch mehrere Stunden dauern, bis ein Verschluss erkannt wird. Die frühe Erkennung eines Verschlusses kann aber bei diesen niedrigen Infusionsraten besonders relevant sein, da häufig hochwirksame Medikamente mit diesen Infusionsraten verabreicht werden. Die Kombination aus niedrigen Infusionsraten und gleichzeitig hochpotenten Medikamenten ist bei den bisherigen auf dem Markt verfügbaren Infusionsgeräten im Fall eines Verschlusses als kritisch zu betrachten.

Ein Problem besteht darin, dass der Einsatz kleinster Druck-Schwellwerte sich bei den konventionellen Methoden besonders schwierig gestaltet, da diese mit einer hohen Fehlalarmquote einhergehen. Grund sind verschiedene Störgrößen, welche das relevante Sensorsignal (Druck/Kraft) überlagern, wie beispielsweise der Übergang von Haft- zur Gleitreibung bei einer Spritzenpumpe (Slip-Stick-Effekt) sowie generelle Schwankungen in der Beschaffenheit der Kunststoffeinmalartikel.

Als eine Alarmbedingung bei einem Verschluss wird typisch der aufgebaute Druck in einem Einmalartikel verwendet, welcher sensorisch über einen Drucksensor ermittelt wird. Als Alarmbedingung kann der Anwender herkömmlich Druckstufen auswählen, welche einen spezifischen Druckwert darstellen. Übersteigt der Druck diesen Druckwert, ist die Alarmbedingung erfüllt und die Pumpe stellt die Förderung ein. Ab dem Zeitpunkt des Verschlusses bis zur Alarmierung liegt jedoch eine Unterförderung vor, welche gerade bei hohen Druckstufen mit niedrigen Förderraten erst nach einer längeren Zeit zur Alarmierung führen.

Der Anwender kann die Sensitivität zur Erkennung einer solchen Unterförderung herkömmlich nur über die Druckstufen regeln und hat somit keinen medizinischen Bezug zum zu wenig geforderten Volumen bzw. Fehlvolumen.

Erfindungsgemäß ist das akzeptierte Fehlvolumen, d.h. der Fehlvolumenschwellenwert, bei einem Verschluss einstellbar. Bei Erreichen des Fehlvolumenschwellenwerts ist die Alarmbedingung erfüllt und es wird ein entsprechender Alarm ausgegeben. Durch Verwendung des Fehlvolumenschwellenwerts als Alarmbedingung anstelle einer herkömmlichen Druckstufe, kann in Verbindung mit der Förderrate bzw. dem eingestellten Volumenstrom auch die verbleibende Zeit bis zu einem Alarm bestimmt werden.

Das fortlaufende Überwachen, ob ein Verschluss der Flüssigkeitsleitung vorliegt, kann beispielsweise mittels Algorithmen, die über maschinelles Lernen trainiert wurden, erfolgen. Basierend auf der Verschlusserkennung kann das Fehlvolumen ab einer hohen Okklusionswahrscheinlichkeit bilanziert werden. Selbstverständlich kann die Verschlusserkennung auch mittels anderer Verfahren erfolgen.

Erfindungsgemäß kann ein Anwender mit ihm bekannten Einheiten (ml etc.) das Fehlvolumen bzw. den Fehlvolumenschwellenwert und damit die Alarmreaktionszeit einstellen. Für kritische Medikamente bzw. zu verabreichende Flüssigkeiten kann ein kleiner Fehlvolumenschwellenwert für eine schnelle Alarmierung eingestellt werden. Für nicht kritische Medikamente kann ein höherer Fehlvolumenschwellenwert eingestellt werden und damit die Verringerung von Alarmen auf einer Station erreicht werden. Für kleine Förderraten kann ein kleinerer Fehlvolumenschwellenwert eingestellt werden, um schneller eine Alarmreaktion zu bewirken.

Der Fehlvolumenschwellenwert kann neben einer Volumeneinheit, beispielsweise in "ml", bei verwendeter Dosisratenkalkulation auch in einer Dosiseinheit wie z.B. "mmol" eingestellt werden. Der Fehlvolumenschwellenwert kann aus einer vom Anwender vorgegebenen Alarmierungszeit kalkuliert werden. Limits und Default-Werte können für den Fehlvolumenschwellenwert festgelegt werden, welche einmalartikelspezifisch oder medikamentenspezifisch über eine Medikamentendatenbank eingespielt werden.

Die Erfindung wird nachfolgend unter Bezugnahme auf die Zeichnungen detailliert beschrieben. Hierbei zeigt:
- Fig. 1: hoch schematisch ein erfindungsgemäßes Infusionssystem,
- Fig. 2: hoch schematisch eine Spritzenpumpe zur Verwendung in einem erfindungsgemäßen Infusionssystem, und
- Fig. 3: hoch schematisch ein gefördertes Volumen der zu verabreichenden Flüssigkeit über der Zeit.

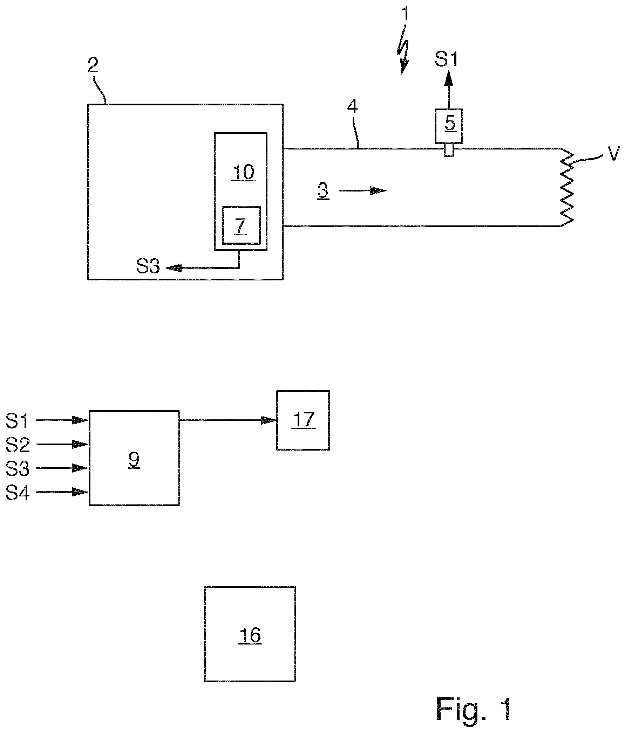

Fig. 1 zeigt hoch schematisch ein erfindungsgemäßes Infusionssystem 1 mit einer herkömmlichen Infusionspumpe 2, die dazu ausgebildet ist, eine zu verabreichende Flüssigkeit 3 in einer Flüssigkeitsleitung 4 zu fördern. Insoweit sei auch auf die einschlägige Fachliteratur verwiesen.

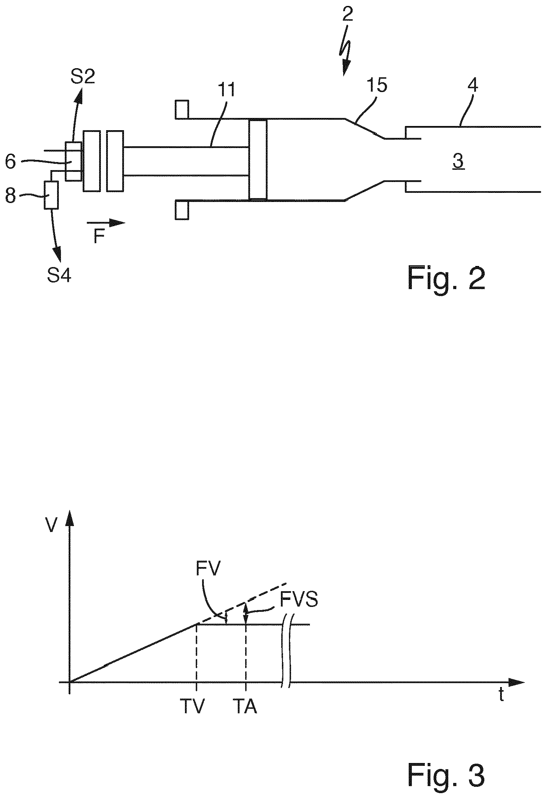

Das Infusionssystem 1 weist weiter Sensoren 5, 6, 7, 8 (siehe auch Fig. 2) zur Erzeugung zugehöriger Sensordaten S1, S2, S3, S4 auf. Die Sensordaten 5, 6, 7, 8 können analoge oder digitale Sensordaten sein.

Das Infusionssystem 1 weist weiter Mittel zur Verschlusserkennung in Form einer KI-Einheit 9 auf, die beispielsweise als Mikroprozessorsystem in Verbindung mit einer geeigneten Software verkörpert sein kann. Die KI-Einheit 9 ist dazu ausgebildet, einen Verschluss V bzw. die Wahrscheinlichkeit des Verschlusses V der Flüssigkeitsleitung 4 in Abhängigkeit von der Anzahl von Sensordaten S1, S2, S3, S4 basierend auf einem statistischen Modell zu ermitteln, wobei das statistische Modell zuvor mittels Trainingsdaten trainiert worden ist. Ein Verschluss kann beispielsweise bei einer ermittelten Verschlusswahrscheinlichkeit von größer als 60 %, 70 % oder 80 % ermittelt werden.

Die Anzahl von Sensoren weist auf: einen Drucksensor 5, der dazu ausgebildet ist, Sensordaten in Form von Druckdaten S1 der zu verabreichenden Flüssigkeit 3 in der Flüssigkeitsleitung 4 zu erzeugen, einen Kraftsensor 6 (siehe Fig. 2), der dazu ausgebildet ist, Sensordaten in Form von Kraftdaten S2 einer mittels der Infusionspumpe 2 erzeugten Pumpkraft F zu erzeugen, einen Motorstromsensor 7, der dazu ausgebildet ist, Sensordaten in Form von Motorstromdaten S3 eines elektrischen Antriebsmotors 10 der Infusionspumpe 2 zu erzeugen, und einen Positionssensor 8 (siehe Fig. 2), der dazu ausgebildet ist, Sensordaten in Form von Positionsdaten S4 eines Spritzenkolbens 11 der Infusionspumpe 2 zu erzeugen.

Die KI-Einheit 9 kann dazu ausgebildet sein, den Verschluss V bzw. dessen Wahrscheinlichkeit weiter in Abhängigkeit von Medikamentendaten, Patientendaten, Daten von weiteren Infusionspumpen, Therapiedaten, Umgebungsbedingungsdaten und/oder Vitalparameterdaten basierend auf dem statistischen Modell zu berechnen.

Das Infusionssystem 1 weist weiter eine Steuereinrichtung 17 auf, die dazu ausgebildet ist, falls ein Verschluss V der Flüssigkeitsleitung mittels der KI-Einheit 9 ermittelt worden ist, ein aufgrund des Verschlusses V der Flüssigkeitsleitung 3 nicht gefördertes Fehlvolumen FV, siehe Fig. 3, der zu verabreichenden Flüssigkeit 3 und/oder eine daraus abgeleitete Größe fortlaufend zu berechnen, und einen Alarm auszugeben, sobald das Fehlvolumen FV der zu verabreichenden Flüssigkeit 3 und/oder die daraus abgeleitete Größe einen einstellbaren Fehlvolumenschwellenwert FVS und/oder einen daraus abgeleiteten Schwellenwert überschreitet.

Fig. 2 zeigt hoch schematisch eine Infusionspumpe 2 in Form einer herkömmlichen Spritzenpumpe zur Verwendung in einem erfindungsgemäßen Infusionssystem 1.

Der Kraftsensor 6 misst die resultierende Kraft F, welche auf eine Kolbenplatte des Spritzenkolbens 11 der Infusionspumpe 2 ausgeübt wird. Diese Kraft F setzt sich zusammen aus der über den Innenquerschnitt der Spritze wirkenden Kraft des (Flüssigkeits-) Drucks und überlagernden Störgrößen. Störgrößen sind unter anderem: positions- und temperaturabhängige Haft/Gleitreibungseffekte, patienten- und therapiespezifische Größen (Durchmesser des Zugangs, Mehrfachinfusion, Art des Zugangs, Bewegung durch Umlagerungen, etc.), physikalische Eigenschaften der zu fördernden Flüssigkeit (Viskosität, Temperatur, etc.) und Effekte verursacht durch Umgebungsbedingungen (Rettungswagen, Hubschrauber, Luftfeuchte, atmosphärischer Druck, etc.).

Im Fall eines Verschlusses steigt der Druck im Verlauf der Förderung an. Die Zunahme des Drucks ist eine Funktion aus der Länge der Flüssigkeitsleitung 4 bis zum Verschluss, d.h. dem Ort des Verschlusses, des verwendeten Materials und Dimensionen des Überleitungssystems, der Temperatur sowie des geförderten Volumens ab dem Zeitpunkt des Verschlusses. Eine steigende Kraft F auf dem Antriebskopf resultiert in einem größeren Motorstrom. Dieser kann alternativ oder zusätzlich zum Kraftsensor mittels des optionalen Motorstromsensors 7 erfasst und ausgewertet werden.

Fig. 3 zeigt hoch schematisch ein gefördertes Volumen V der zu verabreichenden Flüssigkeit 3 über der Zeit t.

Wie dargestellt, wird die zu verabreichende Flüssigkeit 3 bis zum Zeitpunkt TV eines Verschlusses mit konstantem Volumenstrom bzw. konstanter Förderrate gefördert, sodass das Volumen V linear über der Zeit mit einer Steigung zunimmt, die vom eingestellten Volumenstrom bzw. der eingestellten Förderrate abhängt.

Ab dem Zeitpunkt TV des Verschlusses bleibt das geförderte Volumen V konstant. Die Steuereinrichtung 17 berechnet nun, wie oben bereits ausgeführt, das aufgrund des Verschlusses V der Flüssigkeitsleitung 4 nicht geförderte Fehlvolumen FV der zu verabreichenden Flüssigkeit 3. Die Steuereinrichtung 17 gibt zum Zeitpunkt TA einen Alarm aus, sobald das Fehlvolumen FV der zu verabreichenden Flüssigkeit 3 den einstellbaren Fehlvolumenschwellenwert FVS überschreitet.

Der Fehlvolumenschwellenwert FVS kann in Abhängigkeit von der Art der zu verabreichenden Flüssigkeit automatisch eingestellt werden.

Die zu verabreichende Flüssigkeit 3 kann aus einem Einmalartikel 15, vorliegend in Form der Spritze 15, gefördert werden, wobei der Fehlvolumenschwellenwert FVS automatisch eimalartikelspezifisch eingestellt wird.

Der Fehlvolumenschwellenwert FVS kann in Abhängigkeit von einer mit der zu verabreichenden Flüssigkeit 3 korrespondierenden Therapieform, insbesondere automatisch, eingestellt werden. Der Fehlvolumenschwellenwert FVS kann weiter aus einer Medikamentendatenbank 16 ausgelesen werden.

## Patentansprüche

1. Verfahren zum Betreiben eines Infusionssystems (1) mit den Schritten:
- Fördern einer zu verabreichenden Flüssigkeit (3) in einer Flüssigkeitsleitung (4) mit einem vorgebbaren Volumenstrom,
- fortlaufendes Überwachen, ob ein Verschluss (V) der Flüssigkeitsleitung (4) vorliegt,
- falls ein Verschluss der Flüssigkeitsleitung (4) vorliegt, fortlaufendes Berechnen eines aufgrund des Verschlusses (V) der Flüssigkeitsleitung (4) nicht geförderten Fehlvolumens (FV) der zu verabreichenden Flüssigkeit (3) und/oder einer daraus abgeleiteten Größe, und
- Ausgeben eines Alarms, sobald das Fehlvolumen (FV) der zu verabreichenden Flüssigkeit (3) und/oder die daraus abgeleitete Größe einen einstellbaren Fehlvolumenschwellenwert (FVS) und/oder einen daraus abgeleiteten Schwellenwert überschreitet.

2. Verfahren nach Anspruch 1, **dadurch gekennzeichnet, dass**
- der Fehlvolumenschwellenwert (FVS) und/oder der daraus abgeleitete Schwellenwert in Abhängigkeit von der Art der zu verabreichenden Flüssigkeit (3) automatisch eingestellt wird/werden.

3. Verfahren nach einem der vorhergehenden Ansprüche, **dadurch gekennzeichnet, dass**
- die zu verabreichende Flüssigkeit (3) aus einem Einmalartikel (15) gefördert wird, wobei der Fehlvolumenschwellenwert (FVS) und/oder der daraus abgeleitete Schwellenwert automatisch eimalartikelspezifisch eingestellt wird/werden.

4. Verfahren nach einem der vorhergehenden Ansprüche, **dadurch gekennzeichnet, dass**
- der Fehlvolumenschwellenwert (FVS) und/oder der daraus abgeleitete Schwellenwert in Abhängigkeit von einer mit der zu verabreichenden Flüssigkeit (3) korrespondierenden Therapieform, insbesondere automatisch, eingestellt wird/werden.

5. Verfahren nach einem der vorhergehenden Ansprüche, **dadurch gekennzeichnet, dass**
- der Fehlvolumenschwellenwert (FVS) und/oder der daraus abgeleitete Schwellenwert aus einer Medikamentendatenbank (16) ausgelesen wird/werden.

6. Verfahren nach einem der vorhergehenden Ansprüche, **dadurch gekennzeichnet, dass**
- das fortlaufende Überwachen, ob ein Verschluss (V) der Flüssigkeitsleitung (4) vorliegt, die Schritte aufweist:
- Ermitteln einer Anzahl von Sensordaten (S1, S2, S3, S4),
- Speisen einer Kl-Einheit (9) mit der Anzahl von Sensordaten, und
- mittels der KI-Einheit (9) und basierend auf der Anzahl von Sensordaten (S1, S2, S3, S4) Bestimmen, ob ein Verschluss (V) der Flüssigkeitsleitung (4) vorliegt.

7. Verfahren nach Anspruch 6, **dadurch gekennzeichnet, dass**
- die Anzahl von Sensordaten enthält:
- Sensordaten in Form von Druckdaten (S1) der zu verabreichenden Flüssigkeit (3) in der Flüssigkeitsleitung (4), und/oder
- Sensordaten in Form von Kraftdaten (S2) einer mittels einer Infusionspumpe (2) des Infusionssystems (1) erzeugten Pumpkraft (F), und/oder
- Sensordaten in Form von Motorstromdaten (S3) eines elektrischen Antriebsmotors (10) der Infusionspumpe (2), und/oder
- Sensordaten in Form von Positionsdaten (S4) eines Spritzenkolbens (11) der Infusionspumpe (2).

8. Infusionssystem (1), aufweisend:
- Mittel (2) zum Fördern einer zu verabreichenden Flüssigkeit (3) in einer Flüssigkeitsleitung (4) mit einem vorgebbaren Volumenstrom, insbesondere in Form einer Infusionspumpe (2),
- Mittel (9) zum fortlaufenden Überwachen, ob ein Verschluss (V) der Flüssigkeitsleitung (4) vorliegt, insbesondere in Form einer KI-Einheit (9), und
- eine Steuereinrichtung (17), die dazu ausgebildet ist,
- falls ein Verschluss (V) der Flüssigkeitsleitung (4) vorliegt, ein aufgrund des Verschlusses (V) der Flüssigkeitsleitung (3) nicht gefördertes Fehlvolumen (FV) der zu verabreichenden Flüssigkeit (3) und/oder eine daraus abgeleitete Größe fortlaufend zu berechnen, und
- einen Alarm auszugeben, sobald das Fehlvolumen (FV) der zu verabreichenden Flüssigkeit (3) und/oder die daraus abgeleitete Größe einen einstellbaren Fehlvolumenschwellenwert (FVS) und/oder einen daraus abgeleiteten Schwellenwert überschreitet.

9. Infusionssystem (1) nach Anspruch 8, **dadurch gekennzeichnet, dass** das Infusionssystem (1) mindestens einen Sensor der folgenden Sensoren aufweist:
- Drucksensoren (5), die dazu ausgebildet sind, Sensordaten in Form von Druckdaten (S1) der zu verabreichenden Flüssigkeit (3) in der Flüssigkeitsleitung (4) zu erzeugen,
- Kraftsensoren (6), die dazu ausgebildet sind, Sensordaten in Form von Kraftdaten (S2) einer mittels der Infusionspumpe (2) erzeugten Pumpkraft (F) zu erzeugen,
- Motorstromsensoren (7), die dazu ausgebildet sind, Sensordaten in Form von Motorstromdaten (S3) eines elektrischen Antriebsmotors (10) der Infusionspumpe (2) zu erzeugen, und
- Positionssensoren (8), die dazu ausgebildet sind, Sensordaten in Form von Positionsdaten (S4) eines Spritzenkolbens (11) der Infusionspumpe (2) zu erzeugen.
